(19) Europäisches Patentamt
European Patent Office
Office européen des brevets

(11) Publication number: **0 166 583 B1**

(12) # EUROPEAN PATENT SPECIFICATION

(45) Date of publication of patent specification: **18.09.91** (51) Int. Cl.$^5$: **G01N 33/88**

(21) Application number: **85304434.5**

(22) Date of filing: **20.06.85**

(54) **Compositions for enzyme immunoassay of prostaglandins.**

(30) Priority: **28.06.84 JP 131898/84**

(43) Date of publication of application:
**02.01.86 Bulletin 86/01**

(45) Publication of the grant of the patent:
**18.09.91 Bulletin 91/38**

(84) Designated Contracting States:
**AT BE CH DE FR GB IT LI LU NL SE**

(56) References cited:
**EP-A- 0 139 552**
**DE-A- 2 811 537**

**CHEMICAL ABSTRACTS, vol.99, no.3, 18 July 1983, Columbus, OH (US); S.YAMAMOTO et al., p.81, no.16648x**

**CHEMICAL ABSTRACTS, vol.99, no.3, 18 July 1983, Columbus, OH (US); T.INAGAWA et al., pp.81-82, no.16650s**

**PATENT ABSTRACTS OF JAPAN, vol. 10, no. 157 (P-464)[2213], 06 June 1986**

(73) Proprietor: **ONO PHARMACEUTICAL CO., LTD.**
**No. 14, Doshomachi 2-chome Higashi-ku**
**Osaka-shi Osaka(JP)**

(72) Inventor: **Taniguchi, Ken**
**F-108, Shin-ashiyakami 27-ban**
**Suita-shi Osaka(JP)**
Inventor: **Sugiyama, Masayasu**
**26-46, Yamatedai 3-chome**
**Ibaraki-shi Osaka(JP)**
Inventor: **Sawada, Masafumi, 462, Shin-Hakusuimaru-cho**
**Matsuyamachi Higashiiru, Nakadachiuridori**
**Kamigyo-ku, Kyoto-shi, Kyoto(JP)**

(74) Representative: **Pearce, Anthony Richmond et al**
**MARKS & CLERK Alpha Tower Suffolk Street**
**Queensway Birmingham B1 1TT(GB)**

EP 0 166 583 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

1. Field of the Invention

The present invention relates to compositions for the enzyme immunoassay (EIA) of prostaglandins (abbreviated as PGs hereafter) and methods for the determination of PGs by using them. More particularly, the present invention relates to compositions for EIA by means of the Double Antibody Solid Phase method (abbreviated as DASP method hereafter) using PGs as the sample, and methods for determination of PGs by using them.

2. Prior Art

Now, PGs in biological fluids, especially in blood or urine in various diseases have been measured and the relation between diseases and PGs has been discussed (cf. The Japanese Journal of Clinical Medicine, 38, 1139 (1980)). Consequently, methods for the determination of PGs have been improved in order to measure trace amounts of PGs more precisely by more simplified procedures. At present, methods for the determination of PGs are generally classified as follows:

(1) Gas Chromatography-Mass spectrometry method (GC-MS),
(2) Radioimmunoassay using radioactive label (RIA) and
(3) EIA.

Further, EIA includes two methods, ie the Solid Phase Antibody method (or simply called "Solid Phase method" and the Double Antibody Liquid Phase method (or simply called "DA method") (cf. Abstracts on General Meeting of the Japanese Biochemical society in 1983, page 797 (1983) and "Enzyme Immunoassay" the 2nd Edition, edited by Eizi Ishikawa et al, published by Igakushoin (1982) (which is abbreviated as "literature A" hereafter, page 403). Chemical Abstracts 99 (1983), No 16648x (and. Adv. Prostaglandin, Thromboxane, Leukotriene Res. (1983), 11, 181-4) discloses an enzyme immunoassay $TXB_2$ and 6-keto-$PGF_{1a}$ using $\beta$-galactosidase a label. The assay for $TXB_2$ is said to have, as some of its components, $TXB_2$-$\beta$-galactosidase conjugate, rabbit anti-$TXB_2$ serum and goat anti-rabbit IgG antiserum.

Chemical Abstracts (1983) 99, No 16650s (and Adv. Prostaglandin, Thromboxane, Leukotriene Res. 1983, 191-6) discloses an RIA and an EIA for PGE-MUM, wherein the EIA is by a DA method using anti-I serum raised in rabbits.

GC-MS is appreciated as an excellent standard method and RIA is a good method in which relatively many samples can be measured sensitively at the same time, both methods being well known.

In EIA by means of the Solid Phase Antibody method, PGs in a sample can be determined by subjecting PGs in the sample and enzyme-labelled PGs to competitive immuno-reaction for an antibody linked to a support, and then measuring the enzyme activity.

In EIA by means of the Double Antibody Liquid Phase method, PGs in a sample can be determined by subjecting PGs in the sample and enzyme-labelled PGs to competitive immuno-reaction for a first antibody, precipitating by a second antibody, and then measuring the enzyme activity in the precipitates.

On the other hand, EIA by means of the DASP method is also known in the art (cf. "literature A", page 35). In the method, a substance in a sample can be determined by subjecting the substance in the sample and an enzyme-labelkled substance to be measured to competitive immuno-reaction for a first antibody, binding to a second antibody linked to a support, separating the binding substance thus obtained, and then measuring the enzyme activity of the enzyme-labelled actigen bound to the second antibody.

It is known that EIA by means of the DASP method is applicable to the determination of various substances to be measured other than PGs, for example:

(1) the determination of progesterone (cf. Japanese Patent Kokai No 51-91325, British Patent No 1431974, Derwent No 30143X/17),

(2) the determination of an anti-cancer agent (cf. Japanese Patent Kokai No 57-148253, Derwent No 89338E/42),

(3) the determination of crenbuterol (cf. Japanese Patent Kokai No 57-192867, Derwent No 03206K/02 and

(4) the determination of Mytomicin C (cf. Japanese Patent Kokai No. 58-167961, not described in Derwent Abstract.)

Heretofore, there has been no teaching to employ EIA by means of DASP method to the determination of PGs.

3 Problems to be solved

All conventional methods for determination of PGs, however, have great defects and there is no method for determination by which trace of PGs can be measured highly sensitively by a more simplified procedure.

That is, in GC-MS, pretreatment which requires complicated and high technique is essential to clinical application, for example, the extraction of PGs from a sample and the conversion of them into their derivatives suitable for measuring. Therefore, a large quantity of sample is required and many samples can not be measured at the same time. Further, expensive equipment for analysis is required and it is impractical.

In RIA, it is necessary that radioactive isotopes should be treated by specialists in a specially controlled laboratory, and, furthermore, the method has the defect that it is difficult to maintain stable measuring system because the quality of the radio-labelled reagents changes with time.

In EIA by means of the Solid Phase Antibody method, the first antibody which is prepared using expensive and valuable PGs is required in quantity because the first antibody should be linked to a support, and, therefore, the method is not economical. Further, it has the defect that the method is liable to be affected by contaminants in the sample because of competitive reaction between labelled antigen and unlabelled antigen using the first antibody linked to a support, and, therefore, that the assay is insufficiently precise.

In EIA by means of the Double Antibody Liquid Phase method, a large quantity of the second antibody is required in order to separate "bound" from "free" and complicated procedures such as centrifugation for separation are required. Furthermore, it has the defect that the precision is insufficient.

There are some examples that EIA by means of DASP method should be applied to the determination of various substance to be measured other than PGs (cf. item of the DASP method in the above Prior Arts passage. It is a fact that the range and limitation of detection is affected by the type of substance to be measured. It, therefore, cannot be anticipated at all whether or not the range and limitation of detection and assay precision having practical value can be obtained when the method is applied to the determination of PGs.

4. Means to Solve the Problems

Research and development have been carried out in order to establish a method for determination by which trace amounts of the substance (PGs) in a sample can be measured with high sensitivity by more simplified procedures. As a result of energetic investigation of the possibility of PGs being determined by EIA by means of the DASP method, the determination of PGs by the said method has been successful for the first time and it is found to be of practical value. Unexpectedly, it has now been found that EIA by means of the DASP method is surprisingly excellent in the limitation and range of detection and assay precision.

## SUMMARY OF THE INVENTION

Accordingly, an object of the present invention is to provide new compositions for the enzyme immunoassay of a prostaglandin by means of the Double Antibody Solid Phase method.

Another main object of the present invention is to provide methods for determination of prostaglandins by using the said compositions.

## BRIEF DESCRIPTION OF THE DRAWINGS

In the drawings:

Figure 1 is a calibration curve of PGF-MUM,
Figure 2 is a graph showing the correlation between data measured in Example 1 and that by RIA,
Figure 3 is a calibration curve of PGE-MUM,
Figure 4 is a graph showing the correlation between data measured in Example 2 and that by RIA.
Figure 5 is a calibration curve of $TXB_2$,
Figure 6 is a graph showing the correlation between data measured in Example 3 and that by RIA.
Figure 7 is a calibration curve of $LTC_4$, and
Figure 8 is a graph showing the correlation between data measured in Example 4 and that by RIA.

## DETAILED DESCRIPTION OF THE INVENTION

Accordingly, the present invention is concerned with compositions for EIA of PGs which comprises at least the following reagents:

Reagent (A): a first antibody which has been obtained by immunizing a first animal with a prostaglandin conjugated to a protein,

Reagent (B): an antigen consisting of enzyme-labelled PGs, and

Reagent (C): a second antibody, which has been obtained by immunizing a second animal with $\gamma$-globulin or immunoglobulin G from the first animal, linked to a solid phase support,

and methods for determination of PGs by using them.

In the compositions of the present invention, Reagent (A), (B) and (C) hereinbefore described are essential constituents. The present compositions may, if necessary, comprise standard PGs for preparing a calibration curve, a set of reagents required for measuring the activity of labelled enzymes(e.g. substrate, solvent for the substrate and stopper of enzymatic reaction), buffer solutions as subsidiary constituents.

The term PGs as used in the present invention, i.e. PGs which can be determined by using the compositions of the Present invention, refers to derivatives of prostanoic acid having a PC skeleton and to any PGs such that an antibody can be formed when immunizing an animal with the conjugate of a functional group in PGs (e.g. a carboxyl group or an amino group) and a protein. Any PG which forms part of the so-called Arachidonate Cascade and which exists in a stable form may be included. Examples of such PGs are PG compounds, thromboxane (abbreviated as TX hereinafter) compounds and leukotriene (abbreviated as LX hereinafter) compounds, e.g. $PGA_1$, $PGA_2$, $PGA_3$, $PCB_1$, $PGB_2$, $PGB_3$, $PGC_1$, $PGC_2$, $PGC_3$, $PGD_1$, $PGD_2$, $PGD_3$, $PGE_1$, $PGE_2$, $PGE_3$, $PGF_{1\alpha}$, $PGF_{2\alpha}$, $PGF_{3\alpha}$, $PGI_1$, $PGI_2$, $PGI_3$, $TXB_1$, $TXB_2$, $TXB_3$, $LTB_3$, $LTB_4$, $LTB_5$, $LTC_3$, $LTC_4$, $LTC_5$, $LTD_3$, $LTD_4$, $LTD_5$, $LTE_3$, $LTE_4$, $LTE_5$, and their biological metabolites, for example, PGF-MUM (main urinary metabolite of $PGF_{1\alpha}$ or $PGF_{2\alpha}$, having structural formula as shown in Example 1 hereinafter), PGE-MUM (main urinary metabolite of $PGE_1$ or $PGE_2$, having the structural formula as shown in Example 2 hereinafter), 6-keto-$PGF_{1\alpha}$ and 2,3-dinor-6-keto-$PGF_{1\alpha}$ (metabolites of $PGI_2$), 2,3-dinor-$TXB_2$ (metabolite of $TXB_2$). All compounds exemplified herein are, or are considered to be, biological substances or their metabolites. Further, in PGs used in the present invention, derivatives which are prepared by chemical modification of the biological substance described hereinbefore, and their metabolites may be included. Preferred examples of PGs used in the present invention are PGAs, PGEs, PGFs, PGIs, TXBs, LTBs, LTCs, LTDs, LTEs and their metabolites, and more preferable examples of PGs are PGEs, PGFs, TXBs, LTCs and their metabolites, e.g. PGF-MUM, PGE-MUM, 6-keto-$PGF_{1\alpha}$, 2,3-dinor-6-keto-$PGF_{1\alpha}$ and 2,3-dinor-$TXB_2$.

The process for the preparation of reagents constituting the present invention is as follows.

The first antibody of Reagent (A) may be prepared by subjecting a carboxyl group, an amino group or a characterized group newly introduced, of PGs to a conjugation reaction with a characterized group of a protein such as an amino group, a mercapto group, or a hydroxy group, emulsifying the resulting conjugate of PG and protein with an appropriate adjuvant, administering the emulsion to the first animal to immunize, and then collecting serum. Examples of suitable proteins are albumin, globulin, cycloglobulin, hemocyanin and, edestin preferably albumin. The conjugating-reaction of a hapten (i.e. PGs) with a protein is known, and, for example, is described in literature "A", at page 82 in particular. That is, the reaction may be carried out in a suitable solvent (e.g. phosphate buffer), when PGs can be directly conjugated with a protein, by employing a method using a carbodiimide or a method using an acid anhydride, preferably by employing the method using cyclohexylcarbodiimide or using 1-ethyl-3-(3-dimethylaminopropyl)carbodiimide as a conjugating agent, or when PGs can not be directly conjugated with a protein, by employing the method using a spacer known in the field, e.g. 1,5-difluoro-2,4-dinitrobenzene. After reaction, the resulting conjugate may be isolated and purified by column chromatography. Any adjuvants known in this field can be used, for example, complete or incomplete Freund's adjuvant or aluminium hydroxide.

The method for immunization is also known. That is, the immunization is carried out by administering an emulsion of the conjugate of PGs and protein, with the above adjuvant to a suitable animal (e.g. rats, mice, guinea-pigs, rabbits, cats, dogs, sheep and goats) at an appropriate interval for several months. After immunization, serum from the animal may be collected by known methods to obtain the desirable first antibody (Rinshokensa, vol. 26(7), page 777 (1982)) (abbreviated as the literature "B" hereafter).

Practically, many PGs having an analogous chemical structure other than the PGs to be measured also exist in the sample in many cases. Accordingly, it is necessary to obtain an antibody having a high specificity to the PGs to be measured. For example, PGF-MUM has two carboxyl groups. Therefore, an antibody having high specificity to PGF-MUM may be obtained by protecting the carboxyl group in $\alpha$-chain in form or $\delta$-lactone and then conjugating with a protein. An antibody having high specificity to PGE-MUM may be obtained by converting PGE-MUM into the bicyclo compound thereof by alkaline treatment, and then conjugating with a protein. In $LTC_4$, an antibody having high specificity and selectivity cannot be

obtained by the mixed acid anhydride method because of three carboxyl groups in the structure of $LTC_4$ - (cf. structural formula as shown in Example 4 hereinafter). Giving attention to only one amino group in the molecular, an antibody having high selectivity can be obtained by conjugating the amino group with a protein using a spacer.

The antigen of Reagent (B) may be prepared by labelling PGs with an enzyme. That is, the binding of PGs to an enzyme may be carried out by using the conjugation-reaction of PGs with a protein as hereinbefore described in processes for the preparation of Reagent (A). It, preferably, may be carried out by a method using a mixed acid anhydride using isobutylchloroformate or by a method using 4-(N-maleimidomethyl)cyclohexane-1-carboxylic acid N-hydroxysuccineimide ester as a spacer. PGs to be used herein may be those to be measured or any other PGs. Enzymes to be used herein may be any enzymes which are generally used in EIA except for acetylcholine esterase derived from electrophones electricus, for example, malate dehydrogenase, glucose-6-phosphate dehydrogenase, glucose oxidase, peroxidase, acetylcholine esterase from species other than electrophorus electricus, alkaline phosphatase, glucoamylase, lysozyme, β-D-galactosidase, preferably β-D-galactosidase (cf. the literature "A", page 67). In order to enhance the specificity of enzyme-labelled PGs, techniques as hereinbefore described in processes for the preparation of Reagent (A) may be carried out. The sensitivity of determination may, if necessary, be enhanced by changing the quantity of PGs to be bound to an enzyme.

The second antibody of Reagent (C) may be prepared by administering γ-globulin or immunoglobulin G of the same species of animal as used for immunization in preparing the first antibody of Reagent (A), to another species of animal, and then immunizing by generally known methods (cf. the literature "B"). The antibody for the serum of another species of animal is on the market, and may be used. The linkage of the second antibody to a support may be carried out by generally known methods, that is, by physical adsorption or by chemical bond. Any support materials generally used for EIA may be used in the present invention, for example, a polysaccharide such as agarose, dextran and cellulose, a synthetic resin such as polystyrene, glass or polyacrylamide. Any shapes easily separable may be taken for support of the Present invention, for example, small spheres, small test tubes, tubes, fibers, microplates. Polystyrene balls or glass beads are preferred (cf. "Koteika Koso", edited by Ichiro Chihata, published by Kohdansha (1975) in detail).

The standard PGs for preparing a calibration curve, a set of reagents required for measuring the activity of the labelled enzyme and buffer solutions, which may be contained in the compositions of the present invention, may be prepared by methods known per se.

The present invention, furthermore, relates to methods for determination of PGs by using the composition of this invention. The methods for determination may be carried out as follows:

(i) by mixing Reagent (A), Reagent (B) and PGs in a sample in order to effect a competitive antigen-antibody reaction (defined as the first reaction), and adding Reagent (C) to the reaction mixture to bind the antigen-antibody complex to the second antibody adsorbed on the support (defined as the second reaction), and then measuring the enzyme activity of the enzyme-labelled PGs, or

(ii) by subjecting Reagent (A) and PGs in a sample to an antigen-antibody reaction (defined as the first reaction), and adding Reagent (C) to the antigen-antibody complex thus obtained to bind the complex to the second antibody adsorbed on the support (defined as the second reaction), and adding Reagent (B) thereto to bind competitively enzyme-labelled antigen (defined as the third reaction), and then measuring the enzyme activity of enzyme-labelled PGs.

Both methods may be preferably used. It is necessary to use either method depending on the substances to be measured. When PGF-MUM, PGE-MUM or $TXB_2$ is measured, the method (ii) is preferred and when $LTC_4$ is measured, the method (i) is preferred.

The first reaction in the method (i), and the first and second reaction in the method (ii) may be carried out at a temperature from 25° C to 37° C for one to four hours. The sensitivity of determination is not influenced in the range of temperature and time of the reaction hereinbefore described and stable measurement is possible. The second reaction in the method (i) and the third reaction in the method (ii) may be carried out at 4° C overnight.

The method for measuring the enzyme activity nay be carried out by methods known per se (cf. the literature "A", page 21).

(Examples)

The following Examples illustrate, but do not limit, the present invention in detail.

Example 1

Process for the preparation of the composition for measuring PGF-MUM and the measurement of PGF-MUM by using such composition

PGF-MUM has the following structural formula:

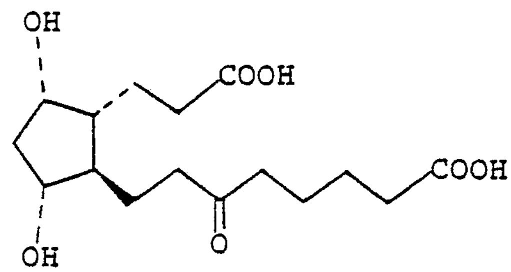

The δ-lactone compound of PGF-MUM has the following structural formula and may be easily prepared chemically.

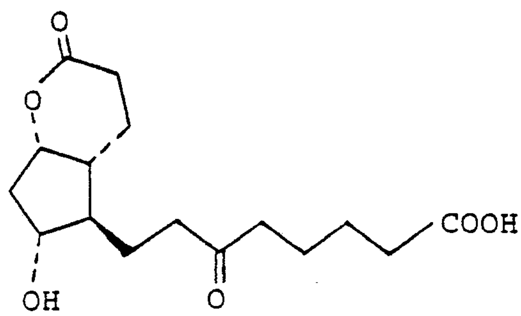

(1) Process for the preparation of the first antibody

4 mg of δ-lactone compound of PGF-MUM and 8 mg of bovine serum albumin (abbreviated as BSA hereafter) were dissolved in each 4 ml of 0.01M phosphate buffer (pH 7.3) and both solutions were mixed. After adding thereto 4 mg of 1-ethyl-3-(3-dimethylaminopropyl) carbodiimide hydrochloride, the mixture was adjusted to pH 5.5 by adding hydrochloric acid and reacted at 4° C for 24 hours under an atmosphere of nitrogen. The reaction mixture thus obtained was dialyzed at 4° C for 24 hours against 0.01M phosphate buffer (pH 7.3) containing 0.15M sodium chloride and then was lyophilized to obtain 8.4 mg of conjugate. The conjugation ratio of PGF-MUM for BSA was calculated by adding $^3$H-PGF-MUM to reaction system and was 10 mol PGF-MUM/mol BSA. 0.5 ml of complete Freund's adjuvant was added to a solution of one mg of the conjugate in 0.5 ml of physiological saline solution and emulsified. The emulsion thus obtained was administered intradermally to dorsal skin and paws of male Japanese white rabbits to immunize. The immunization was carried out six times at two-week intervals and blood was collected from the carotid artery after two weeks from the latest immunization. Blood thus obtained was allowed to stand for 30 minutes at room temperature and the serum was collected by centrifugation to obtain the first antibody. The first antibody thus obtained was stored at -20° C after lyophilization.

(2) Process for the preparation of PGF-MUM labelled by β-D-galactosidase (abbreviated as FMO hereafter)

A mixture of 2.5 μl of tri-n-butylamine in 25 μl of dioxane, 1.3 μl of isobutylchloroformate in 25 μl of dioxane and 2 mg of δ-lactone compound of PGF-MUM in 50 μl of dioxane was reacted at 10° C for 30 minutes. The reaction mixture was added dropwise at 10° C over 10 minutes to a solution of 0.4 mg of β-D-galactosidase (manufactured by Boehringer Mannheim) in 0.7 ml of 0.5% sodium bicarbonate. The solution thus obtained was allowed to stand at 4° C for two hours under an atmosphere of nitrogen and purified by Sephadex G-25® column (manufactured by Pharmacia) (1.0 x 20 cm) to obtain FMO.

(3) Process for the preparation of the second antibody linked to a support (abbreviated as DASP ball)

IgG (i.e. immunoglobulin G) fraction of the second antibody was prepared by employing DEAE-cellulose column chromatography and $Na_2SO_4$ fraction from sheep anti rabbit $\gamma$-globulin antiserum (prepared by Daiichi Radioisotope) (Cf. Men-eki Jikken Sohsa-hou, edited by the Japanese Immunology Association, vol. IV, page 1137 (1975)). 3000 polystyrene balls were soaked in 500 ml of 0.05M phosphate buffer (pH 7.4) containing 1 mg/ml of the second antibody IgG and were allowed to stand for two hours at room temperature and then overnight at 4° C for absorption to obtain desired DASP ball.

(4) Process for the preparation of compositions (kits) for measuring PGF-MUM (100 tests per kit)

A kit comprises the following reagents:

(a) Buffer Solution:

250 ml of 30 mM phosphate buffer (pH 6.8) containing 0.3M sodium chloride, 3 mM magnesium chloride, 0.3% BSA and 0.3% of sodium azide. The solution should be diluted with water three times prior to use (defined as "Buff-A" hereafter).

(b) Standard PGF-MUM:

A solution of 500 ng of PGF-MUM dissolved in one ml of Buff-A solution from which BSA is removed.

(C) The First Antibody:

60 ml of a solution that the first antibody prepared in (1) hereinbefore described was diluted with Buf-A solution 400,000 times.

(d) FMO:

12 ml of Buff-A solution containing FMO prepared in (2) hereinbefore described in concentration of 60 ng/ml.

(e) DASP Ball:

100 DASP ball prepared in (3) hereinbefore described, soaked in 100 ml of Buff-A solution.

(f) Substrate of Enzyme:

Two vials in which 4-methylumbelliferyl-$\beta$-D-galactoside was dissolved in 12 ml of 10 mM phosphate buffer (pH 7.0) containing 0.5% mannite, being in concentration of 0.5 mM, and then the obtained solution was lyophilyzed. The contents should be diluted with 30 ml off Buff-A solution to be in concentration of 0.2 mM prior to use.

(g) Stopper of Enzymatic Reaction:

30 ml of 1 M glycine buffer (pH 10.5). The solution should be diluted with water ten times prior to use.

(5) Measurement of PGF-MUM by using a kit prepared in (4)

Frozen urine was thawed and centrifuged for ten minutes (3000 rpm). Its supernatant was used as an urine sample.

0.5 ml of the first antibody (reagent (c) in the kit) was added to each 20 $\mu$l of the urine sample or standard PGF-MUM having various concentrations (reagent (b) in the kit) and the solution was reacted for one hour at 37° C (defined as the first reaction). DASP balls (reagent (e) in the kit) were added to the first reaction mixture (one DASP ball/sample) and reacted for two hours at 37° C (defined as the second reaction). 0.1 ml of FMO (reagent (d) in the kit) was added to the second reaction mixture containing DASP ball and the mixture was reacted overnight at 4° C (defined as the third reaction). After reaction, the supernatant was discarded and the remaining DASP ball was washed twice with 2.5 ml of Buff-A solution (reagent (a) in the kit) and then the ball was reacted in 0.5 ml off the substrate of enzyme (reagent (f) in the kit) for 30 to 60 minutes at 37° C in order to measure the activity off $\beta$-D-galactosidase bound to DASP ball. The reaction was stopped by adding 2.5 ml of the stopper of enzymatic reaction (reagent (g) in the kit). Fluorescence intensity of the produced 4-methylumbelliferone was measured. The wavelengths fixed for excitation and emission was 360 and 450 nm, respectively. The calibration curve is shown in Figure 1.

It was found that PGF-MUM in urine can be measured in the range of 10 to 5000 pg by using the composition of the present invention.

The correlation between the concentration of PGF-MUM measured in Example 1 (Y) (50 samples) and that measured by the RIA method (X) (described in Prostaglandins, vol. 10, page 549 - 555 (1975)) is shown in Figure 2.

$$Y = 1.1713X + 6.3864$$

Correlation coefficient: $\gamma = 0.9606$

These values shows that the correlation between the two methods is excellent.

The concentration of PGF-MUM in urine of healthy human, measured by using the composition of Example 1 was in the range from 3.1 to 60.0 ng/ml (mean value: 24.1 ± 13.2 ng/ml, n = 21).

Example 2

Process for the preparation of the composition for measuring PGE-MUM and the measurement of PGE-MUM by using such composition

PGE-MUM has the following structural formula:

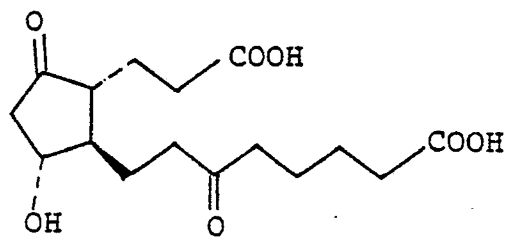

PGE-MUM may be easily converted into the stable bicyclo compound of PGE-MUM having the following structural formula by alkaline treatment.

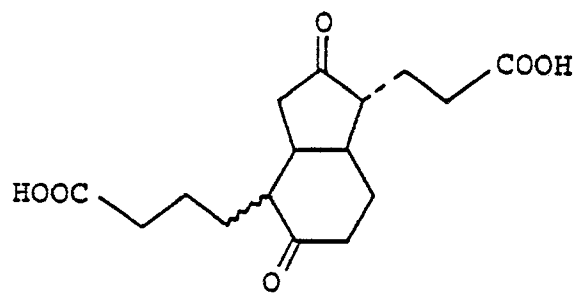

(1) Process for the preparation of the first antibody

By the same procedure as described in Example 1-(1), the first antibody was prepared by using 4 mg of the bicyclo compound of PGE-MUM and 6 mg of BSA. The conjugation ratio of PGE-MUM for BSA was 10 mol bicyclo compound of PGE-MUM/mol BSA.

(2) Process for the preparation of PGE-MUM labelled by β-D-galactosidase (abbreviated as EMO hereafter)

By the same procedure as described in Example 1-(2), EMO was prepared by using 100 μg of the bicyclo compound of PGE-MUM and 0.4 mg of β-D-galactosidase.

(3) Process for the preparation of the second antibody linked to a support (abbreviated as DASP ball)

DASP ball was prepared by the same procedure as described in Example 1-(3).

(4) Process for the preparation of compositions (kits) for measuring PGE-MUM (100 tests per kit)

A kit comprises the following reagents:

(a) Buffer solution-1:
250 ml of the buffer solution prepared in Example 1-(4)-(a). The solution should be diluted with water three times prior to use (defined as "Buf-A" hereafter).
(b) Buffer solution-2:
500 ml of 50 mM phosphate buffer (pH 7.0) containing 0.1% sodium azide.
(c) Buffer solution-3:
100 ml of Buff-A solution containing 0.05% gelatin.

(d) Solution for conversion:
10 ml of 4M(4N) sodium hydroxide solution.
(e) Solution for neutralization:
10 ml of 4M(4N) hydrochloric acid
(f) Standard PGE-MUM:
A solution of 500 ng of the bicyclo compound of PGE-MUM dissolved in one ml of Buff-A solution from which BSA is removed.
(g) The First Antibody:
60 ml of a solution that the first antibody prepared in (1) hereinbefore described was diluted with Buf-A solution 300,000 times.
(h) EMO:
12 ml of Buff-A solution containing EMO prepared in (2) hereinbefore described, in concentration of 500 ng/ml.
(i) DASP Ball:
DASP ball prepared by the same procedure as described in Example 1-(4)-(e).
(j) Substrate of Enzyme:
Substrate of enzyme prepared by the same procedure as described in Example 1-(4)-(f).
(k) Stopper of Enzymatic Reaction:
Stopper prepared by the same procedure as described in Example 1-(4)-(g).

(5) Measurement of PGE-MUM by using a kit prepared in (4)

As pre-treatment, to 0.1 ml of the supernatant obtained by thawing the freezed urine and then by centrifuging, was added 0.1 ml of the solution for conversion (reagent (d) in the kit) and the obtained solution was reacted for one hour at room temperature. To the reaction solution was added 0.1 ml of the solution for neutralization (reagent (e) in the kit) and the obtained solution was diluted with 5 ml of the buffer solution-2 (reagent (b) in the kit) to obtain an urine sample.

0.5 ml of the first antibody (reagent (g) in the kit) was added to each 0.1 ml of the urine sample or standard PGE-MUM having various concentration (reagent (f) in the kit) and the mixture was reacted for one hour at 37° C (defined as the first reaction). DASP balls (reagent (i) in the kit) were added to the first reaction mixture (one DASP ball/sample) and reacted for two hours at 37° C (defined as the second reaction). 0.1 ml off EMO (reagent (h) in the kit) was added to the second reaction mixture containing DASP ball and the mixture was reacted overnight at 4° C (defined as the third reaction). After reaction, the supernatant was discarded and the remaining DASP ball was washed twice with 2.5 ml of Buff-A solution (reagent (a) in the kit) and then the activity of β-D-galactosidase bound to DASP ball was measured by the same procedure as described in Example 1-(5). The calibration curve is shown in Figure 3.

It was found that the bicyclo compound of PGE-MUM in urine can be measured in the range of 0.5 to 2000 pg by using the composition of the present invention.

The correlation between the concentration of the bicyclo compound of PGE-MUM measured in Example 2 (Y) (39 samples) and that measured by the RIA method (X) (described in Advance in Prostaglandins, Thromboxane, and Leukotriene Research, vol. 11, page 191 - 196 (1983)) is shown in Figure 4.

$$Y = 1.247X + 4.395$$

Correlation coefficient: $\gamma$ = 0.9841
These values shows that the correlation between the two methods is excellent.

Example 3

Process for the preparation off the composition for measuring $TXB_2$ and the measurement of $TXB_2$ by using such composition
$TXB_2$ has the following structural formula:

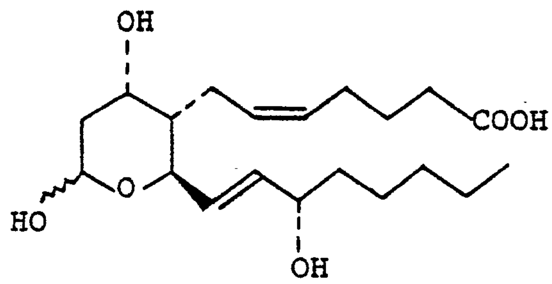

(1) Process for the preparation of the first antibody

By the same procedure as described in Example 1-(1), the first antibody was prepared by using 4 mg of $TXB_2$ and 8 mg of BSA. The conjugation ratio of $TXB_2$ for BSA was 9.4 mol $TXB_2$/mol BSA.

(2) Process for the preparation of $TXB_2$ labelled by $\beta$-D-galactosidase (abbreviated as TBG hereafter)

By the same procedure as described in Example 1-(2), TBG was prepared by using 250 $\mu$g of $TXB_2$ and 0.4 mg of $\beta$-D-galactosidase.

(3) Process for the preparation of the second antibody linked to a support (abbreviated as DASP ball)

After about 1000 glass beads were soaked in the detergent containing proteolytic enzyme overnight for washing enough, they were dried for 5 hours at 500° C. The beads thus obtained were soaked in 100 ml of acetone containing 2% 3-aminopropyltriethoxysilane, allowed to stand for 24 hours at 45° C, and then washed with acetone several times and dried at room temperature. The beads thus obtained were soaked in 1% aqueous solution of glutaraldehyde allowed to stand for one hour at room temperature, and then washed enough with water and 0.25M phosphate buffer (pH 7.5), successively. The beads thus obtained were soaked in 100 ml of 0.25M phosphate buffer (pH 7.5) containing 13 mg of the second antibody IgG (prepared in Example 1-(3)), and were allowed to stand overnight at 4° C for absorption to obtain desired DASP ball.

(4) Process for the preparation of compositions (kits) for measuring $TXB_2$ (100 tests per kit).

A kit comprises the following reagents:
(a) Buffer solution:
250 ml of the buffer solution prepared in Example 1-(4)-(a). The solution should be diluted with water three times prior to use (defined as "Buf-A" hereafter).
(b) Standard $TXB_2$:
A solution off 500 ng of $TXB_2$ dissolved in one ml of Buff-A solution from which BSA is removed.
(c) the First Antibody:
60 ml of a solution that the first antibody prepared in (1) hereinbefore described was diluted with Buf-A solution 200,000 times.
(d) TBG:
12 ml of Buf-A solution containing TBG prepared in (2) hereinbefore described, in concentration of 120 ng/ml.
(E) DASP Ball:
100 DASP ball prepared in (3) hereinbefore described, soaked in 100 ml of Buf-A solution.
(f) Substrate of Enzyme:
Substrate of enzyme prepared by the same procedure as described in Example 1-(4)-(f).
(g) Stopper of Enzymatic Reaction:
Stopper prepared by the same procedure as described in Example 1-(4)-(g).

(5) Measurement of $TXB_2$ by using a kit prepared in (4)

Abdominal ascites of rats from which blood cell was removed by centrifugation, were diluted with Buf-A

solution (reagent (a) in the kit) 10 times to be used as an sample.

0.5 ml of the first antibody (reagent (c) in the kit) was added to each 0.1 ml of the sample or standard $TXB_2$ having various concentration (reagent (b) in the kit) and the mixture was reacted for one hour at 37° C (defined as the first reaction). DASP balls (reagent (e) in the kit) were added to the first reaction mixture (one DASP ball/sample) and reacted for two hours at 37° C (defined as the second reaction). 0.1 ml of TBG (reagent (d) in the kit) was added to the second reaction mixture containing DASP ball and the mixture was reacted overnight at 4° C (defined as the third reaction). After reaction, the supernatant was discarded and the remaining DASP ball was washed twice with 2.5 ml of Buf-A solution (reagent (a) in the kit) and then the activity of β-D-galactosidase bound to DASP ball was measured by the same procedure as described in Example 1-15). The calibration curve is shown in Figure 5.

It was found that $TXB_2$ in abdominal ascites can be measured in the range of 2 to 1000 pg by using the composition of the present invention.

The correlation between the concentration of $TXB_2$ measured in Example 3 (Y) (8 samples) and that measured by the RIA method (X) is shown in Figure 6.

$$Y = 0.996X + 0.430$$

Correlation coefficient: $\gamma = 0.9947$

These value shows that the correlation between two methods is excellent.

Example 4

Process for the preparation of the composition for measuring $LTC_4$ and the measurement of $LTC_4$ by using such composition

$LTC_4$ has the following structural formula:

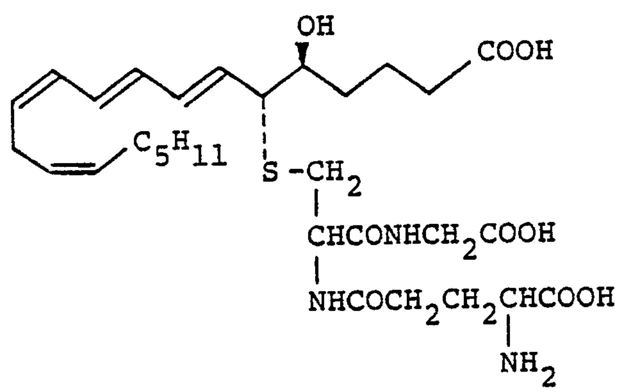

(1) Process for the preparation of the first antibody

A solution of 10 mg of 1,5-difluoro-2,4-dinitrobenzene (DFDNB) in 0.7 ml of methanol was added to a solution of 2.5 mg of $LTC_4$ in 0.5 ml of 0.1M phosphate buffer (pH 7.2, degassed and replaced by argon gas) and the mixture was stirred for 30 minutes at 24° C. After reaction, methanol was removed by using argon gas and unreacted DFDNB was removed from the reaction mixture by the extraction with diethyl ether (0.5 ml x 3 times). After diethyl ether remaining in the solution was removed by using argon gas, a solution of 10 mg of BSA in one ml of 0.2M borate buffer (pH 8.5, degassed and replaced by argon gas) was added thereto, and the solution thus obtained was allowed to stand for two days at room temperature under an atmosphere of argon with shading the light. After reaction, the reaction mixture was purified by Sephadex G-25® column (manufactured by Pharmacia) (1.0 x 50 cm) with shading the light by using distilled water (degassed and replaced by argon gas) as eluent to obtain a protein fraction. The obtained fraction was lyophilized to obtain 9.8 mg of conjugate. The conjugation ratio of $LTC_4$ for BSA was 8.14 mol $LTC_4$/mol BSA. One ml of incomplete Freund's adjuvant was added to a solution of one mg of the conjugate thus obtained in 0.5 ml of physiological saline solution and emulsified under an atmosphere of argon. The obtained emulsion was administered subcutaneously to the dorsal neck of rabbits at 15 points to immunize. The immunization was carried out four times at three-week intervals and blood was collected after 10 days

from the latest immunization and then the serum was collected by centrifugation to obtain the first antibody.

(2) Process for the preparation of $LTC_4$ labelled by β-D-galactosidase (abbreviated as LCG hereafter)

A solution of one mg of 4-(N-maleimidomethyl)-cyclohexane-1-carboxylic acid N-hydroxysuccinimide ester (MCAE) in 0.1 ml of tetrahydrofuran was added to a solution of 100 μg of $LTC_4$ in one ml of 0.075M phosphate buffer (pH 7.2, degassed and replaced by argon gas) and the mixture was reacted for 30 minutes at 24° C under an atmosphere of argon. After reaction, tetrahydrofuran was removed by using argon gas and unreacted MCAE was removed from the reaction mixture by the extraction with diethyl ether (1 ml x 3 times). Diethyl ether remaining in the aqueous layer was completely removed by using argon gas to obtain cyclohexylmaleimide derivative of $LTC_4$. The derivative thus obtained was added dropwise to a solution of 1.5 mg of β-D-galactosidase in one ml of 0.075M phosphate buffer (pH 7.2, degassed and replaced by argon gas) under an atmosphere of argon. After addition the mixture was stirred for 30 minutes at 24° C and the reaction mixture thus obtained was purified by Sephadex G-25® column (manufactured by Pharmacia) (1.0 x 50 cm) to obtain LCG.

(3) Process for the preparation of the second antibody linked to a support (abbreviated as DASP ball)

DASP ball was prepared by the same procedure as described in Example 1-(3).

(4) Process for the preparation of compositions (kits) for measuring $LTC_4$ (100 tests per kit)
A kit comprises the following reagents:

(a) Buffer solution:
250 ml of the buffer solution prepared in Example 1-(4)-(a). The solution should be diluted with water three times prior to use (defined as "Buf-A" hereafter).
(b) Standard $LTC_4$:
A solution of 500 ng of $LTC_4$ dissolved in one ml of Buf-A solution (degassed and replaced by argon gas) from which BSA is removed.
(c) the First Antibody:
60 ml of a solution that the first antibody prepared in (1) hereinbefore described was diluted with Buf-A solution 15,000 times.
(d) LCG:
12 ml of Buf-A solution (degassed and replaced by argon gas) containing 1800 ng of LCG prepared in (2) hereinbefore described.
(e) DASP Ball:
DASP ball prepared by the same procedure as described in Example 1-(4)-(e).
(f) Substrate of Enzyme:
Substrate of enzyme prepared by the same procedure as described in Example 1-(4)-(f).
(g) Stopper of Enzymatic Reaction:
Stopper prepared by the same procedure as described in Example 1-(4)-(g).

(5) Measurement of $LTC_4$ by using a kit prepared in (4)

Abdominal ascites of rats pretreated by the same procedure as described in Example 3-(5) were used as a sample.

0.5 ml of the first antibody (reagent (c) in the kit) and 0.1 ml of LCG (reagent (d) in the kit) were added to each 0.1 ml of the sample or standard $LTC_4$ having various concentration (reagent (b) in the kit). The mixture was reacted for one hour at 25° C (defined as the first reaction). DASP balls (reagent (e) in the kit) were added to the first reaction mixture (one DASP ball/sample) and reacted overnight at 4° C. After reaction, the supernatant was discarded and the remaining DASP ball was washed twice with 2.5 ml of Buf-A solution (reagent (a) in the kit) and then the activity of β-D-galactosidase bound to DASP ball was measured by the same procedure as described in Example 1-(5). The calibration curve is shown in Figure 7.

It was found that $LTC_4$ in abdominal ascites can be measured in the range of 10 to 5000 pg by using the composition of the present invention.

The correlation between the concentration of $LTC_4$ measured in Example 4 (Y) (12 samples) and that measured by the RIA method (X) is shown in Figure 8.

Y = 0.980X + 22.42

Correlation coefficient: $\gamma$ = 0.979

These values shows that the correlation between the two methods is excellent.

Effects of the Invention

As can be understood from the Examples, trace amounts of the substance in samples can be measured by more simplified procedure by using the compositions for measurement of the present invention. Furthermore, it has now been found that the method of the present invention is surprisingly excellent in the range and limitation of detection, and assay precision.

The method of the present invention can be compared with conventional methods in the range and limitation of detection, and assay precision as shown in the following Table 1.

TABLE 1

| | | Range of Detection (pg) | Limitation of Detection (pg) | Assay Precision[1)] (Ratio S/N) |
|---|---|---|---|---|
| Present method | Measurement in Example 1 (PGF-MUM) | 10 - 5000 | 10 | 1255/45 = 27.9 |
| | Measurement in Example 2 (PGE-MUM) | 0.5 - 2000 | 0.5 | 1200/30 = 40 |
| | Measurement in Example 3 ($TXB_2$) | 2 - 1000 | 2 | 74.5/14.9 = 5 |
| | Measurement in Example 4 ($LTC_4$) | 10 - 5000 | 10 | 2236/83 = 26.9 |
| Conventional method | Measurement by the RIA method (PGF-MUM) | 20 - 2000 | 20 | 11369/241 = 47 |
| | Measurement by the RIA method (PGE-MUM) | 4 - 4000 | 4 | 11877/290 = 41 |
| | Measurement by the RIA method ($TXB_2$) | 5 - 250 | 5 | 7500/400 = 18.8 |
| | Measurement by the RIA method ($LTC_4$) | 50 - 5000 | 50 | 723/78 = 9.3 |
| | Measurement by the Double Antibody Liquid Phase method (PGF-MUM) | 10 - 6500 | 10 | 5250/396 = 13.3 |
| | Measurement by the Double Antibody Liquid Phase method (PGE-MUM) | 0.5 - 8000 | 0.5 | 1335/644 = 2.1 |
| | Measurement by the DASP method (Pepleomycin)[2)] | 100 - 200000 | 100 | --- |
| | Measurement by the DASP method (Crenbuterol)[3)] | 0.125 - 64 | 0.125 | --- |
| | Measurement by the DASP method (Mytomicin C)[4)] | 1000 - 50000 | 1000 | --- |

1) Ratio S/N means ratio Signal/Noise and is generally used as a guide for assay precision. It is understood that the greater ratio S/N is, the better assay precision is.

2) These values are described in Japanese Patent Kokai No. 57-148253 (Derwent No. 89338E/42).

3) These values are described in Japanese Patent Kokai No. 57-192867 (Derwent No. 03206K/02).

4) These values are described in Japanese Patent Kokai No. 58-167961.

As understood from the Table, the determination by the method of the present invention:
(i) is superior to that by RIA by a factor of 2 to 8 in the limitation of detection,
(ii) is superior to that by the Double Antibody Liquid Phase method by a factor 2 to 20 in assay precision, and
(iii) has the almost same level of the range and limitation of detection as the determination by the DASP method using crenbuterol as a sample, and moreover is highly superior to that by the DASP method

using pepleomycin or mytomicin C as a sample in the range and limitation of detection. Some examples that the DASP method may be applied to the determination of some substances to be measured other than those shown in the Table, are already known. However, it is a fact that the range and limitation of detection should be greatly affected by the type of substance to be measured.

Apart from the above, the determination by the method of the present invention has the following characteristics.

(1) The pretreatment is simpler as compared with GC-MS, and many samples can be measured at the same time.

(2) The treatment by specialists in a specially controlled laboratory,as required in RIA, is unnecessary, and the enzyme-labelled reagent is stable for a long time.

(3) The first antibody is not required in as great a quantity as that required in the Solid Phase Antibody method and, therefore, it is economical. Further, the present method is hardly affected by contaminants in the sample.

(4) The present method has wider applicability than both the Solid Phase Antibody method and the Double Antibody Liquid Phase method because the second antibody for the first antibody should be linked to a solid phase support. That is simply by changing the type of first antibody, compositions for measuring various PGs may be obtained by using in common the second antibody linked to a solid phase support, i.e. Reagent (C).

(5) A large quantity of the second antibody is not required as compared with the Double Antibody Liquid Phase method,and it is easy to separate "bound" from "free" (No complicated procedures such as centrifugation are required.)

(6) The Double Antibody Solid Phase method is superior to the Double Antibody Liquid Phase method in reproducibility, and it is possible to develop the autoanalytic system. For example, procedures such as the washing and movement of balls can be easily carried out by burying a magnet in the ball. Furthermore, when the second antibody is linked to each well of a microplate, it is possible to effect immuno-reaction in the said wells and to measure the enzyme activity automatically per well.

**Claims**

**Claims for the following Contacting States: BE CH DE FR GB IT LI LU NL SE:**

**1.** A composition for the enzyme immunoassay of a prostaglandin which comprises at least the following reagents:

Reagent (A): a first antibody which has been obtained by immunizing a first animal with a prostaglandin conjugated to a protein,

Reagent (B): an antigen consisting of an enzyme-labelled prostaglandin, and

Reagent (C): a second antibody, which has been obtained by immunizing a second animal with gamma-globulin or immunoglobulin G from the first animal, linked to a solid phase support.

**2.** A composition according to claim 1, which further comprises:

Reagent (D): a standard prostaglandin

Reagent (E): a set of reagents required for measuring the activity of the labelled enzyme, and

Reagent (F): buffer solutions.

**3.** A composition according to claim 1 or 2, wherein the prostaglandin is selected from prostaglandin A, E, F and I compounds, thromboxane B compounds, Leukotriene B, C, D and E compounds, and their biological metabolites.

**4.** A composition according to claim 3, wherein the prostaglandin is selected from prostaglandin E and F compounds, thromboxane B compounds, Leukotriene C compounds, and their biological metabolites.

**5.** A composition according to claim 1 or 2, wherein the enzyme is $\beta$-D-galactosidase.

**6.** A composition for the enzyme immunoassay of the main urinary metabolite of prostaglandin $E_1$ or $E_2$ - (abbreviated as PGE-MUM hereafter), which comprises at least the following reagents:

Reagent (A): a first antibody which has been obtained by immunizing a first animal with a bicyclo compound of PGE-MUM conjugated to bovine serum albumin,

Reagent (B): an antigen consisting of a bicyclo compound of PGE-MUM labelled with $\beta$-D-

galactosidase, and

Reagent (C): a second antibody, which has been obtained by immunizing a second animal with gamma-globulin or immunoglobulin G from the first animal, linked to a solid phase support.

**7.** A composition for the enzyme immunoassay of the main urinary metabolite of prostaglandin $F_{1\alpha}$ or $F_{2\alpha}$ - (abbreviated as PGF-MUM hereafter), which comprises at least the following reagents:

Reagent (A): a first antibody which has been obtained by immunizing a first animal with a $\delta$-lactone compound of PGF-MUM conjugated to bovine serum albumin,

Reagent (B): an antigen consisting of a $\delta$-lactone compound of PGF-MUM labelled with $\beta$-D-galactosidase, and

Reagent (C): a second antibody, which has been obtained by immunizing a second animal with gamma-globulin or immunoglobulin G from the first animal, linked to a solid phase support.

**8.** A composition for the enzyme immunoassay of thromboxane $B_2$, which comprises at least the following reagents:

Reagent (A): a first antibody which has been obtained by immunizing a first animal with thromboxane $B_2$ conjugated to bovine serum albumin,

Reagent (B): an antigen consisting of thromboxane $B_2$ labelled with $\beta$-D-galactosidase, and

Reagent (C): a second antibody, which has been obtained by immunizing a second animal with gamma-globulin or immunoglobulin G from the first animal, linked to a solid phase support.

**9.** A composition for the enzyme immunoassay of leukotriene $C_4$, which comprises at least the following reagents:

Reagent (A): a first antibody which has been obtained by immunizing a first animal with leukotriene $C_4$ conjugated to bovine serum albumin,

Reagent (B): a antigen consisting of leukotriene $C_4$ labelled with $\beta$-D-galactosidase, and

Reagent (C): a second antibody, which has been obtained by immunizing a second animal with gamma-globulin or immunoglobulin G from the first animal, linked to a solid phase support.

**10.** A method for the determination of a prostaglandin by enzyme immunoassay which utilises the composition of Claim 1, which assay is characterized by mixing Reagent (A), Reagent (B) and a prostaglandin in a sample in order to effect a competitive antigen-antibody reaction; adding thereto Reagent (C) to bind the antigen-antibody complex to the second antibody linked to the support; and then measuring the enzyme activity of the enzyme-labelled prostaglandin which is bound to the support.

**11.** A method for the determination of a prostaglandin by enzyme immunoassay, which is characterized by subjecting Reagent (A) (as defined in Claim 1) and a prostaglandin in a sample to an antigen-antibody reaction, adding Reagent (C) (as defined in Claim 1) to the antigen-antibody complex thus obtained to bind the complex to the second antibody linked to the support; adding thereto Reagent (B) (as defined in Claim 1) to bind competitively enzyme-labelled antigen; and then measuring the enzyme activity of the enzyme-labelled prostaglandin which is bound to the support.

**Claims for the following Contracting State: AT:**

**1.** A method of producing a composition for the enzyme immunoassay of a prostaglandin which comprises at least Reagents (A), (B) and (C), said method comprising obtaining a first antibody (Reagent (A)) by immunizing a first animal with a prostaglandin conjugated to a protein; obtaining an antigen consisting of an enzyme-labelled prostaglandin (Reagent (B)); and obtaining Reagent (C) by immunizing a second animal with gamma-globulin or immunoglobulin G from the first animal to produce a second antibody and linking the latter to a solid phase support.

**2.** A method according to Claim 1, further comprising the step of assembling Reagents (A) to (C) with:

Reagent (D): a standard prostaglandin

Reagent (E): a set of reagents required for measuring the activity of the labelled enzyme, and
Reagent (F): buffer solutions.

**3.** A method according to Claim 1 or 2, wherein the prostaglandin is selected from prostaglandin A, E, F and I compounds, thromboxane B compounds, Leukotriene B, C, D and E compounds, and their biological metabolites.

**4.** A method according to Claim 3, wherein the prostaglandin is selected from prostaglandin E and F compounds, thromboxane B compounds, Leukotriene C compounds, and their biological metabolites.

**5.** A method according to Claim 1 or 2, wherein the enzyme is $\beta$-D-galactosidase.

**6.** A method of producing a composition for the enzyme immunoassay off the main urinary metabolite of prostaglandin $E_1$ or $E_2$ (abbreviated as PGE-MUM hereafter), said composition comprising at least Reagents (A), (B), and (C), said method comprising obtaining a first antibody (Reagent (A)) by immunizing a first animal with a bicyclo compound of PGE-MUM conjugated to bovine serum albumin; obtaining an antigen consisting of a bicyclo compound of PGE-MUM labelled with $\beta$-D-galactosidase (Reagent (B)); and obtaining Reagent (C) by immunizing a second animal with gamma-globulin or immunoglobulin G from the first animal to produce a second antibody and linking the latter to a solid phase support.

**7.** A method of producing a composition for the enzyme immunoassay of the main urinary metabolite of prostaglandin $F_{1\alpha}$ or $F_{2\alpha}$ (abbreviated as PGF-MUM hereafter), said composition comprising Reagents (A), (B) and (C), said method comprising obtaining a first antibody (Reagent (A)) by immunizing a first animal with a $\delta$-lactone compound of PGF-MUM conjugated to bovine serum albumin; obtaining an antigen consisting of a $\delta$-lactone compound of PGF-MUM labelled with $\beta$-D-galactosidase (Reagent (B)-); and obtaining Reagent (C) by immunizing a second animal with gamma-globulin or immunoglobulin G from the first animal to produce a second antibody and linking the latter to a solid phase support.

**8.** A method of producing a composition for the enzyme immunoassay of thromboxane $B_2$, said composition comprising at least the following reagents: Reagents (A), (B) and (c), said method comprising obtaining a first antibody (Reagent (A)) by immunizing a first animal with thromboxane $B_2$ conjugated to bovine serum albumin; obtaining an antigen consisting of thromboxane $B_2$ labelled with $\beta$-D-galactosidase (Reagent (B)); and obtaining Reagent (C) by immunizing a second animal with gamma-globulin or immunoglobulin G from the first animal to produce a second antibody and linking the latter to a solid phase support.

**9.** A method of producing a composition for the enzyme immunoassay of leukotriene $C_4$, said composition comprising at least the following reagents: Reagents (A), (B) and (C), said method comprising obtaining a first antibody (Reagent (A)) by immunizing a first animal with leukotriene $C_4$ conjugated to bovine serum albumin; obtaining a antigen consisting of leukotriene $C_4$ labelled with $\beta$-D-galactosidase (Reagent (B)); and obtaining Reagent (C) by immunizing a second animal with gamma-globulin or immunoglobulin G from the first animal to produce a second antibody and linking the latter to a solid phase support.

**10.** A method for the determination of a prostaglandin by enzyme immunoassay which utilises a composition as defined in Claim 1, which assay is characterized by mixing Reagent (A), Reagent (B) and a prostaglandin in a sample in order to effect a competitive antigen-antibody reaction; adding thereto Reagent (C) to bind the antigen-antibody complex to the second antibody linked to the support; and then measuring the enzyme activity off the enzyme-labelled prostaglandin which is bound to the support.

**11.** A method for the determination of a prostaglandin by enzyme immunoassay, which is characterized by subjecting Reagent (A) (as defined in Claim 1) and a prostaglandin in a sample to an antigen-antibody reaction, adding Reagent (C) (as defined in Claim 1) to the antigen-antibody complex thus obtained to bind the complex to the second antibody linked to the support; adding thereto Reagent (B) (as defined in Claim 1) to bind competitively enzyme-labelled antigen; and then measuring the enzyme activity of the enzyme-labelled prostaglandin which is bound to the support.

**Revendications**

**Revendications pour les Etats contractants suivants: BE, CH, DE, FR, GB, IT, LI, LU, NL, SE**

**1.** Composition pour l'essai immuno-enzymatique d'une prostaglandine qui comprend au moins les réactifs suivants :

réactif (A) : un premier anticorps que l'on a obtenu par immunisation d'un premier animal avec une prostaglandine conjuguée à une protéine,

réactif (B) : un antigène constitué d'une prostaglandine à marquage enzymatique, et

réactif (C) : un second anticorps que l'on a obtenu par immunisation d'un second animal avec une $\gamma$-globuline ou une immunoglobuline G du premier animal, lié à un support en phase solide.

**2.** Composition selon la revendication 1 qui comprend de plus :

réactif (D) : un standard de prostaglandine

réactif (E) : un ensemble de réactifs nécessaires pour mesurer l'activité de l'enzyme marquée, et

réactif (F) : des solutions tampons.

**3.** Composition selon la revendication 1 ou 2, dans laquelle la prostaglandine est choisie parmi les composés de type prostaglandine A, E, F et I, les composés de type thromboxane B, les composés de type leucotriène B, C, D et E et leurs métabolites biologiques.

**4.** Composition selon la revendication 3, dans laquelle la prostaglandine est choisie parmi les composés de type prostaglandine E et F, les composés de type thromboxane B, les composés de type leucotriène C et leurs métabolites biologiques.

**5.** Composition selon la revendication 1 ou 2, dans laquelle l'enzyme est la $\beta$-D-galactosidase.

**6.** Composition pour l'essai immuno-enzymatique du métabolite urinaire principal des prostaglandines $E_1$ ou $E_2$ (désigné ci-après par l'abréviations PGE-MUM), qui comprend au moins les réactifs suivants :

réactif (A) : un premier anticorps que l'on a obtenu par immunisation d'un premier animal avec un dérivé bicyclo de PGE-MUM conjugué à de la sérum-albumine bovine,

réactif (B) : un antigène constitué d'un dérivé bicyclo de PGE-MUM marqué avec la $\beta$-D-galactosidase, et

réactif (C) : un second anticorps que l'on a obtenu par immunisation d'un second animal avec une $\gamma$-globuline ou une immunoglobuline G du premier animal, lié à un support en phase solide.

**7.** Composition pour l'essai immuno-enzymatique du métabolite urinaire principal des prostaglandines $F_{1\alpha}$ ou $F_{2\alpha}$ (désigné ci-après par l'abréviations PGF-MUM), qui comprend au moins les réactifs suivants :

réactif (A) : un premier anticorps que l'on a obtenu par immunisation d'un premier animal avec une $\delta$-lactone de PGF-MUM conjuguée à de la sérum-albumine bovine,

réactif (a) : un antigène constitué d'une $\delta$-lactone de PGF-MUM marquée avec la $\beta$-D-galactosidase, et

réactif (C) : un second anticorps que l'on a obtenu par immunisation d'un second animal avec une $\gamma$-globuline ou une immunoglobuline G du premier animal, lié à un support en phase solide.

**8.** Composition pour l'essai immuno-enzymatique du thromboxane $B_2$, qui comprend au moins les réactifs suivants :

réactif (A) : un premier anticorps que l'on a obtenu par immunisation d'un premier animal avec du thromboxane $B_2$ conjugué à de la sérum-albumine bovine,

réactif (B) : un antigène constitué de thromboxane $B_2$ marqué avec la $\beta$-D-galactosidase, et

réactif (C) : un second anticorps que l'on a obtenu par immunisation d'un second animal avec une $\gamma$-globuline ou une immunoglobuline G du premier animal, lié à un support en phase solide.

**9.** Composition pour l'essai immuno-enzymatique du leucotriène $C_4$, qui comprend au moins les réactifs suivants :

réactif (A) : un premier anticorps que l'on a obtenu par immunisation d'un premier animal avec le leucotriène $C_4$ conjugué à de la sérum-albumine bovine,
réactif (B) : un antigène constitué de leucotriène $C_4$ marqué avec la $\beta$-D-galactosidase, et
réactif (C) : un second anticorps que l'on a obtenu par immunisation d'un second animal avec une $\gamma$-globuline ou une immunoglobuline G du premier animal, lié à un support en phase solide.

**10.** Procédé pour la détermination d'une prostaglandine par essai immuno-enzymatique qui utilise la composition de la revendication 1, lequel essai est caractérisé par le mélange du réactif (A), du réactif (B) et d'une prostaglandine dans un échantillon pour effectuer une réaction compétitive antigène-anticorps ; l'addition du réactif (C) pour lier le complexe antigène-anticorps au second anticorps lié au support ; puis la mesure de l'activité enzymatique de la prostaglandine à marquage enzymatique qui est liée au support.

**11.** Procédé pour la détermination d'une prostaglandine par essai immuno-enzymatique, qui est caractérisé en ce que l'on soumet le réactif (A) (comme défini dans la revendication 1) et une prostaglandine dans un échantillon à une réaction antigène-anticorps, on ajoute le réactif (C) (comme défini dans la revendication 1) au complexe antigène-anticorps ainsi obtenu pour lier le complexe au second anticorps lié au support ; on ajoute le réactif (B) (comme défini dans la revendication 1) pour lier compétitivement l'antigène à marquage enzymatique ; puis on mesure l'activité enzymatique de la prostaglandine à marquage enzymatique qui est liée au support.

**Revendications pour l'Etat contractant Suivant: AT**

**1.** Procédé pour produire une composition pour l'essai immuno-enzymatique d'une prostaglandine, qui comprend au moins les réactifs (A), (B) et (C), ledit procédé comprenant l'obtention d'un premier anticorps (réactif (A)) par immunisation d'un premier animal avec une prostaglandine conjuguée à une protéine ; l'obtention d'un antigène constitué d'une prostaglandine à marquage enzymatique (réactif (B)) ; et l'obtention du réactif (C) par immunisation d'un second animal avec une $\gamma$-globuline ou une immunoglobuline G du premier animal pour produire un second anticorps et la liaison de ce dernier à un support en phase solide.

**2.** Procédé selon la revendication 1 comprenant de plus l'étape de réunion des réactifs (A) à (C) avec :
réactif (D) : un standard de prostaglandine
réactif (E) : un ensemble de réactifs nécessaire pour mesurer l'activité de l'enzyme marquée, et
réactif (F) : des solutions tampons.

**3.** Procédé selon la revendication 1 ou 2, dans lequel la prostaglandine est choisie parmi les composés de type prostaglandine A, E, F et I, les composés de type thromboxane B, les composés de type leucotriène B, C, D et E et leurs métabolites biologiques.

**4.** Procédé selon la revendication 3, dans lequel la prostaglandine est choisie parmi les composés de type prostaglandine E et F, les composés de type thromboxane B, les composés de type leucotriène C et leurs métabolites biologiques.

**5.** Procédé selon la revendication 1 ou 2, dans lequel l'enzyme est la $\beta$-D-galactosidase.

**6.** Procédé pour produire une composition pour l'essai immuno-enzymatique du métabolite urinaire principal des prostaglandines $E_1$ ou $E_2$ (désigné ci-après par l'abréviation PGE-MUM), ladite composition comprenant au moins les réactifs (A), (B) et (C), ledit procédé comprenant l'obtention d'un premier anticorps (réactif (A)) par immunisation d'un premier animal avec un dérivé bicyclo de PGE-MUM conjugué à de la sérum-albumine bovine ; l'obtention d'un antigène constitué d'un dérivé bicyclo de PGE-MUM marqué avec la $\beta$-D-galactosidase (réactif (B)) ; et l'obtention du réactif (C) par immunisation d'un second animal avec une $\gamma$-globuline ou une immunoglobuline G du premier animal pour produire un second anticorps, et la liaison de ce dernier à un support en phase solide.

**7.** Procédé pour produire une composition pour l'essai immuno-enzymatique du métabolite urinaire principal des prostaglandines $F_{1\alpha}$ ou $F_{2\alpha}$ (désigné ci-après par l'abréviation PGF-MUM), ladite composition comprenant les réactifs (A), (B) et (C), ledit procédé comprenant l'obtention d'un premier anticorps (réactif (A)) par immunisation d'un premier animal avec une δ-lactone de PGF-MUM conjuguée à de la sérum-albumine bovine ; l'obtention d'un antigène constitué d'une δ-lactone de PGF-MUM marquée avec la β-D-galactosidase (réactif (B)) ; et l'obtention du réactif (C) par immunisation d'un second animal avec une γ-globuline ou une immunoglobuline G du premier animal pour produire un second anticorps, et la liaison de ce dernier à un support en phase solide.

**8.** Procédé pour produire une composition pour l'essai immuno-enzymatique du thromboxane $B_2$, ladite composition comprenant au moins les réactifs suivants : réactifs (A), (B) et (C), ledit procédé comprenant l'obtention d'un premier anticorps (réactif (A)) par immunisation d'un premier animal avec le thromboxane $B_2$ conjugué à de la sérum-albumine bovine ; l'obtention d'un antigène constitué de thromboxane $B_2$ marqué avec la β-D-galactosidase (réactif (B)) ; et l'obtention du réactif (C) par immunisation d'un second animal avec une γ-globuline ou une immunoglobuline G du premier animal pour produire un second anticorps, et la liaison de ce dernier à un support en phase solide.

**9.** Procédé pour produire une composition pour l'essai immuno-enzymatique du leucotriène $C_4$, ladite composition comprenant au moins les réactifs suivants : réactifs (A), (B) et (C), ledit procédé comprenant l'obtention d'un premier anticorps (réactif (A)) par immunisation d'un premier animal avec un leucotriène $C_4$ conjugué à de la sérum-albumine bovine ; l'obtention d'un antigène constitué d'un leucotriène $C_4$ marqué avec la β-D-galactosidase (réactif (B)) ; et l'obtention du réactif (C) par immunisation d'un second animal avec une γ-globuline ou une immunoglobuline G du premier animal pour produire un second anticorps, et la liaison de ce dernier à un support en phase solide.

**10.** Procédé pour la détermination d'une prostaglandine par essai immuno-enzymatique qui utilise la composition de la revendication 1, lequel essai est caractérisé par le mélange du réactif (A), du réactif (B) et d'une prostaglandine dans un échantillon pour effectuer une réaction compétitive antigèneanticorps ; l'addition du réactif (C) pour lier le complexe antigène-anticorps au second anticorps lié au support ; puis la mesure de l'activité enzymatique de la prostaglandine à marquage enzymatique qui est liée au support.

**11.** Procédé pour la détermination d'une prostaglandine par essai immuno-enzymatique, qui est caractérisé en ce que l'on soumet le réactif (A) (comme défini dans la revendication 1) et une prostaglandine dans un échantillon à une réaction antigène-anticorps, on ajoute le réactif (C) (comme défini dans la revendication 1) au complexe antigène-anticorps ainsi obtenu pour lier le complexe au second anticorps lié au support ; on ajoute le réactif (B) (comme défini dans la revendication 1) pour lier compétitivement l'antigène à marquage enzymatique ; puis on mesure l'activité enzymatique de la prostaglandine à marquage enzymatique qui est liée au support.

**Patentansprüche**

**Patentansprüche für folgende Vertragsstaaten: BE, CH, DE, FR, GB, IT, LI, LU, NL, SE**

**1.** Zusammensetzung für das Enzym-Immun-Assay eines Prostaglandins, welche zumindest die folgenden Reagenzien enthält:
Reagens (A): einen ersten Antikörper, der durch Immunisieren eines ersten Tieres mit einem mit Protein konjugierten Prostaglandin hergestellt wurde,
Reagens (B): ein Antigen bestehend aus einem Enzymmarkierten Prostaglandin, und
Reagens (C): einen zweiten Antikörper, der durch Immunisieren eines zweiten Tieres mit Gammaglobulin oder Immunglobulin G aus dem ersten Tier hergestellt wurde, gebunden an einen Festphasen-Träger.

**2.** Zusammensetzung nach Anspruch 1, welche weiters enthält: Reagens (D): ein Standardprostaglandin, Reagens (E): einen für das Messen der Aktivität des markierten Enzyms erforderlichen Reagenziensatz, und Reagens (F): Pufferlösungen.

**3.** Zusammensetzung nach Anspruch 1 oder 2, dadurch gekennzeichnet, daß das Prostaglandin ausgewählt wird aus Prostaglandin A-, E-, F- und I-Verbindungen, Thromboxan B-Verbindungen, Leukotrien B-, C-, D- und E-Verbindungen, und ihren biologischen Metaboliten.

4. Zusammensetzung nach Anspruch 3, dadurch gekennzeichnet, daß das Prostaglandin ausgewählt wird aus Prostaglandin E- und F-Verbindungen, Thromboxan B-Verbindungen, Leukotrien C-Verbindungen, und ihren biologischen Metaboliten.

5. Zusammensetzung nach Anspruch 1 oder 2, dadurch gekennzeichnet, daß das Enzym $\beta$-D-Galactosidase ist.

6. Zusammensetzung für das Enzym-Immun-Assay des Hauptmetaboliten von Prostaglandin $E_1$ oder $E_2$ im Harn (im weiteren als PGE-MUM abgekürzt), welche zumindest die folgenden Reagenzien enthält:
Reagens (A): einen ersten Antikörper, der durch Immunisieren eines ersten Tieres mit einer bicyclischen Verbindung von PGE-MUM konjugiert mit Rinderserumalbumin hergestellt wurde,
Reagens (B): ein Antigen bestehend aus einer bicyclischen Verbindung von PGE-MUM markiert mit $\beta$-D-Galactosidase, und
Reagens (C): einen zweiten Antikörper, der durch Immunisieren eines zweiten Tieres mit Gammaglobulin oder Immunglobulin G aus dem ersten Tier hergestellt wurde, gebunden an einen Festphasen-Träger.

7. Zusammensetzung für das Enzym-Immun-Assay des Hauptmetaboliten von Prostaglandin $F_{1\alpha}$ oder $F_{2\alpha}$ im Harn (im weiteren als PGF-MUM abgekürzt), welche zumindest die folgenden Reagenzien enthält:
Reagens (A): einen ersten Antikörper, der durch Immunisieren eines ersten Tieres mit einer $\delta$-Lactonverbindung von PGF-MUM konjugiert mit Rinderserumalbumin hergestellt wurde,
Reagens (B): ein Antigen bestehend aus einer $\delta$-Lactonverbindung von PGF-MUM markiert mit $\beta$-D-Galactosidase, und
Reagens (C): einen zweiten Antikörper, der durch Immunisieren eines zweiten Tieres mit Gammaglobulin oder Immmunglobulin G aus dem ersten Tier hergestellt wurde, gebunden an einen Festphasen-Träger.

8. Zusammensetzung für das Enzym-Immun-Assay von Thromboxan $B_2$, welche zumindest die folgenden Reagenzien enthält:
Reagens (A): einen ersten Antikörper, der durch Immunisieren eines ersten Tieres mit Thromboxan $B_2$ konjugiert mit Rinderserumalbumin hergestellt wurde,
Reagens (B): ein Antigen bestehend aus Thromboxan $B_2$ markiert mit $\beta$-D-Galactosidase, und
Reagens (C): einen zweiten Antikörper, der durch Immunisieren eines zweiten Tieres mit Gammaglobulin oder Immunglobulin G aus dem ersten Tier hergestellt wurde, gebunden an einen Festphasen-Träger.

9. Zusammensetzung für das Enzym-Immun-Assay von Leukotrien $C_4$, welche zumindest die folgenden Reagenzien enthält: Reagens (A): einen ersten Antikörper, der durch Immunisieren eines ersten Tieres mit Leukotrien $C_4$ konjugiert mit Rinderserumalbumin hergestellt wurde, Reagens (B): ein Antigen bestehend aus Leukotrien $C_4$ markiert mit $\beta$-D-Galactosidase, und Reagens (C): einen zweiten Antikörper, der durch Immunisieren eines zweiten Tieres mit Gammaglobulin oder Immunglobulin G aus dem ersten Tier hergestellt wurde, gebunden an einen Festphasen-Träger.

10. Verfahren zur Bestimmung eines Prostaglandins durch Enzym-Immun-Assay, welches die Zusammensetzung des Anspruchs 1 verwendet, wobei das Assay dadurch gekennzeichnet ist, daß Reagens (A), Reagens (B) und ein Prostaglandin in einer Probe gemischt werden, um eine kompetitive Antigen-Antikörper-Reaktion zu bewirken; Reagens (c) beigegeben wird, um den Antigen-Antikörper-Komplex an den zweiten, an den Träger gebundenen Antikörper zu binden; und dann die Enzymaktivität des Enzymmarkierten Prostaglandins, das an den Träger gebunden ist, gemessen wird.

11. Verfahren zur Bestimmung eines Prostaglandins durch Enzym-Immun-Assay, dadurch gekennzeichnet, daß Reagens (A) (wie in Anspruch 1 definiert) und ein Prostaglandin in einer Probe einer Antigen-Antikörper-Reaktion unterworfen wird, Reagens (C) (wie in Anspruch 1 definiert) dem so erhaltenen Antigen-Antikörper-Komplex beigegeben wird, um den Komplex an den zweiten, an den Träger gebundenen Antikörper zu binden; Reagens (B) (wie in Anspruch 1 definiert) beigegeben wird, um Enzym-markiertes Antigen kompetitiv zu binden; und dann die Enzymaktivität des Enzym-markierten Prostaglandins, das an den Träger gebunden ist, gemessen wird.

**Pantentanspruch für folgende Vertragsstaaten: AT**

1. Verfahren zur Herstellung einer Zusammensetzung für das Enzym-Immun-Assay eines Prostaglandins, welche zumindest die Reagenzien (A), (B) und (C) enthält, wobei dieses Verfahren umfaßt: Herstellen eines ersten Antikörpers (Reagens (A)) durch Immunisieren eines ersten Tieres mit einem Prostaglandin konjugiert mit einem Protein; Herstellen eines Antigens bestehend aus einem Enzym-markierten Prostaglandin (Reagens (B)), und Herstellen von Reagens (C) durch Immunisieren eines zweiten Tieres mit Gammaglobulin oder Immunglobulin G aus dem ersten Tier, um einen zweiten Antikörper zu erzeugen, und Binden des letzteren an einen Festphasen-Träger.

2. Verfahren nach Anspruch 1, weiters umfassend das Verbinden der Reagenzien (A) bis (C) mit:
Reagens (D): ein Standardprostaglandin
Reagens (E): einen für das Messen der Aktivität des markierten Enzyms erforderlichen Reagenziensatz, und
Reagens (F): Pufferlösungen

3. Verfahren nach Anspruch 1 oder 2, dadurch gekennzeichnet, daß das Prostaglandin ausgewählt wird aus Prostaglandin A-, E-, F- und I-Verbindungen, Thromboxan B-Verbindungen, Leukotrien B-, C-, D- und E-Verbindungen, und ihren biologischen Metaboliten.

4. Verfahren nach Anspruch 3, dadurch gekennzeichnet, daß das Prostaglandin ausgewählt wird aus Prostaglandin E- und F-Verbindungen, Thromboxan B-Verbindungen, Leukotrien C-Verbindungen, und ihren biologischen Metaboliten.

5. Verfahren nach Anspruch 1 oder 2, dadurch gekennzeichnet, daß das Enzym $\beta$-D-Galactosidase ist.

6. Verfahren zur Herstellung einer Zusammensetzung für das Enzym-Immun-Assay des Hauptmetaboliten von Prostaglandin $E_1$ oder $E_2$ im Harn (im weiteren abgekürzt als PGE-MUM), wobei diese Zusammensetzung zumindest die Reagenzien (A), (B) und (C) enthält, und wobei dieses Verfahren umfaßt: Herstellen eines ersten Antikörpers (Reagens (A)) durch Immunisieren eines ersten Tieres mit einer bicyclischen Verbindung von PGE-MUM konjugiert mit Rinderserumalbumin; Herstellen eines Antigens bestehend aus einer bicyclischen Verbindung von PGE-MUM markiert mit $\beta$-D-Galactosidase (Reagens (B)); und Herstellen von Reagens (C) durch Immunisieren eines zweiten Tieres mit Gammaglobulin oder Immunglobulin G aus dem ersten Tier, um einen zweiten Antikörper zu erzeugen, und Binden des letzteren an einen Festphasen-Träger.

7. Verfahren zur Herstellung einer Zusammensetzung für das Enzym-Immun-Assay des Hauptmetaboliten von Prostaglanding $F_{1\alpha}$ oder $F_{2\alpha}$ im Harn (im weiteren abgekürzt als PGF-MUM), wobei diese Zusammensetzung die Reagenzien (A), (B) und (C) enthält, und wobei dieses Verfahren umfaßt: Herstellen eines ersten Antikörpers (Reagens (A)) durch Immunisieren eines ersten Tieres mit einer $\delta$-Lactonverbindung von PGF-MUM konjugiert mit Rinderserumalbumin; Herstellen eines Antigens bestehend aus einer $\delta$-Lactonverbindung von PGF-MUM markiert mit $\beta$-D-Galactosidase (Reagens (B)); und Herstellen von Reagens (C) durch Immunisieren eines zweiten Tieres mit Gammaglobulin oder Immunglobulin G aus dem ersten Tier, um einen zweiten Antikörper zu erzeugen, und Binden des letzteren an einen Festphasen-Träger.

8. Verfahren zur Herstellung einer Zusammensetzung für das Enzym-Immun-Assay von Thromoxan $B_2$, wobei diese Zusammensetzung zumindest die folgenden Reagenzien enthält: Reagens (A), (B) und (C), und wobei dieses Verfahren umfaßt: Herstellen eines ersten Antikörpers (Reagens (A)) durch Immunisieren eines ersten Tieres mit Thromboxan $B_2$ konjugiert mit Rinderserumalbumin; Herstellen eines Antigens bestehend aus Thromboxan $B_2$ markiert mit $\beta$-D-Galactosidase (Reagens (B)); und Herstellen von Reagens (C) durch Immunisieren eines zweiten Tieres mit Gammaglobulin oder Immunglobulin G aus dem erten Tier, um einen zweiten Antikörper zu erzeugen, und Binden des letzteren an einen Festphasen-Träger.

9. Verfahren zur Herstellung einer Zusammensetzung für das Enzym-Immun-Assay von Leukotriene $C_4$, wobei diese Zusammensetzung zumindest die folgenden Reagenzien enthält: Reagens (A), (B) und (C)-,und wobei dieses Verfahren umfaßt: Herstellen eines ersten Antikörpers (Reagens (A)) durch Immunisieren eines ersten Tieres mit Leukotrien $C_4$ konjugiert mit Rinderserumalbumin; Herstellen eines Antigens bestehend aus Leukotrien $C_4$ markiert mit $\beta$-D-Galactosidase (Reagens (B)); und Herstellen von Reagens (C) durch Immunisieren eines zweiten Tieres mit Gammaglobulin oder Immunglobulin G aus dem ersten Tier, um einen zweiten Antikörper zu erzeugen, und Binden des letzteren an einen Festphasen-Träger.

**10.** Verfahren zur Bestimmung eines Prostaglandins durch Enzym-Immun-Assay, welches die im Anspruch 1 definierte Zusammensetzung verwendet, wobei das Assay dadurch gekennzeichnet ist, daß Reagens (A), Reagens (B) und ein Prostaglandin in einer Probe gemischt werden, um eine kompetitive Antigen-Antikörper-Reaktion zu bewirken; Reagens (C) beigegeben wird, um den Antigen-Antikörper-Komplex an den zweiten, an den Träger gebundenen Antikörper zu binden; und dann die Enzymaktivität des Enzym-markierten Prostaglandins, das an den Träger gebunden ist, gemessen wird.

**11.** Verfahren zur Bestimmung eines Prostaglandins durch Enzym-Immun-Assay, dadurch gekennzeichnet, daß Reagens (A) (wie in Anspruch 1 definiert) und ein Prostaglandin in einer Probe einer Antigen-Antikörper-Reaktion unterworfen wird, Reagens (C) (wie in Anspruch 1 definiert) dem so erhaltenen Antigen-Antikörper-Komplex beigegeben wird, um den Komplex an den zweiten, an den Träger gebundenen Antikörper zu binden; Reagens (B) (wie in Anspruch 1 definiert) beigegeben wird, um Enzym-markiertes Antigen kompetitiv zu binden; und dann die Enzymaktivität des Enzym-markierten Prostaglandins, das an den Träger gebunden ist, gemessen wird.

Figure 1

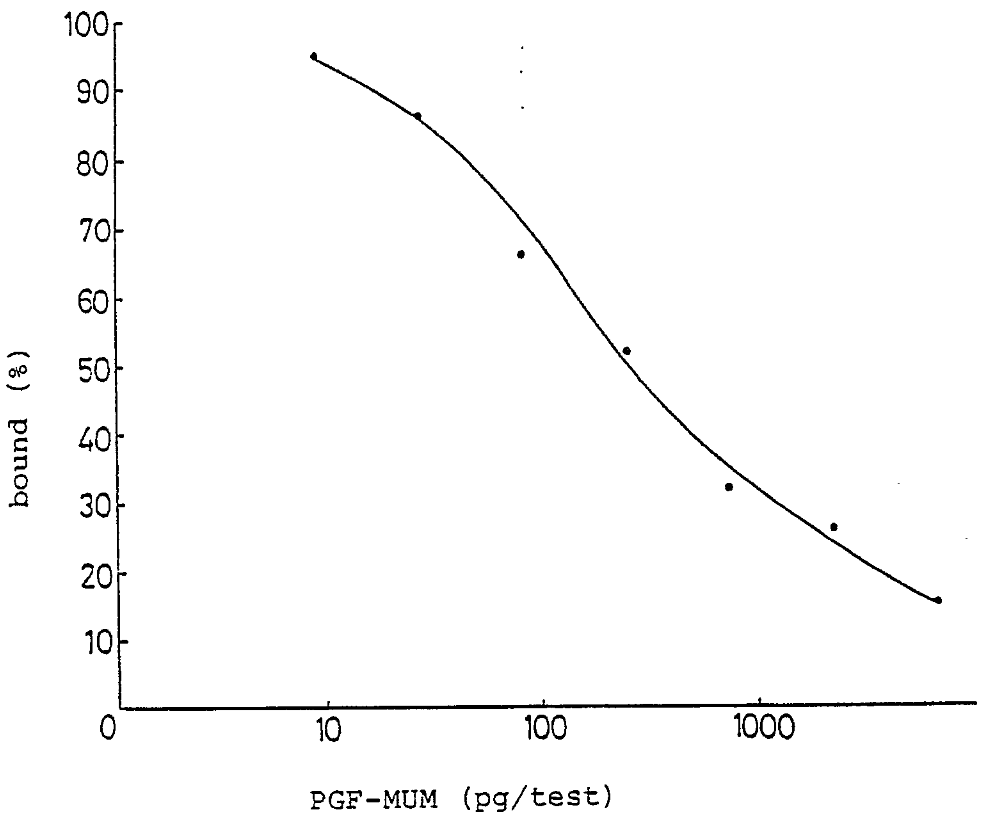

Figure 2

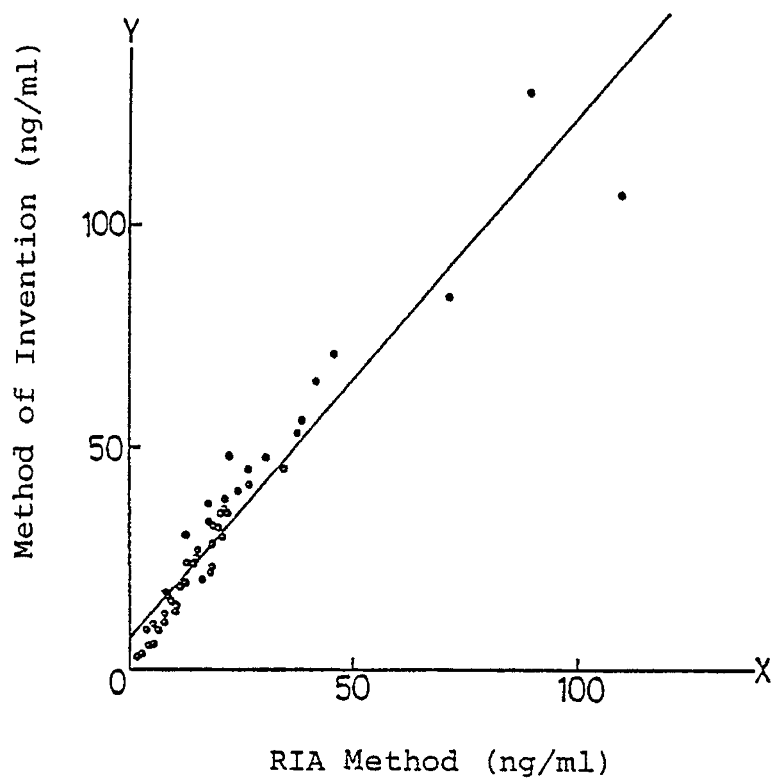

Figure 3

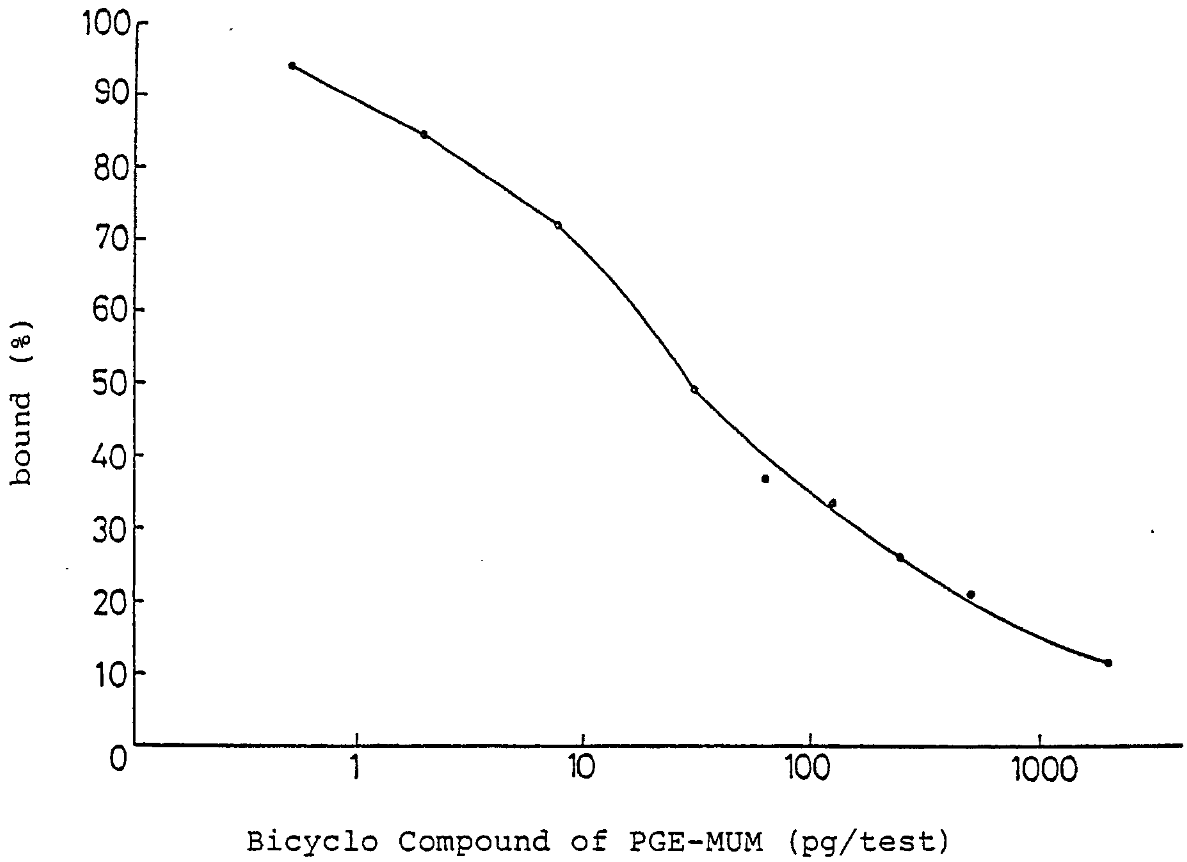

100
90
80
70
60
50
40
30
20
10
0
bound (%)
1
10
100
1000
Bicyclo Compound of PGE-MUM (pg/test)

Figure 4

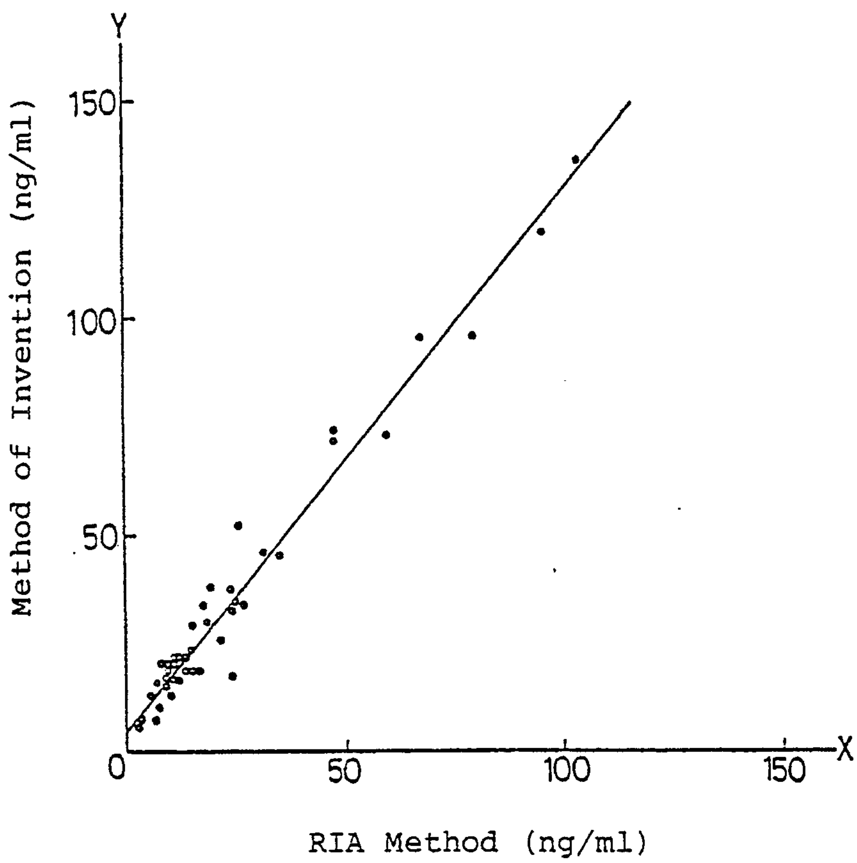
Y
150
100
50
0
50
100
150
X
Method of Invention (ng/ml)
RIA Method (ng/ml)

Figure 5

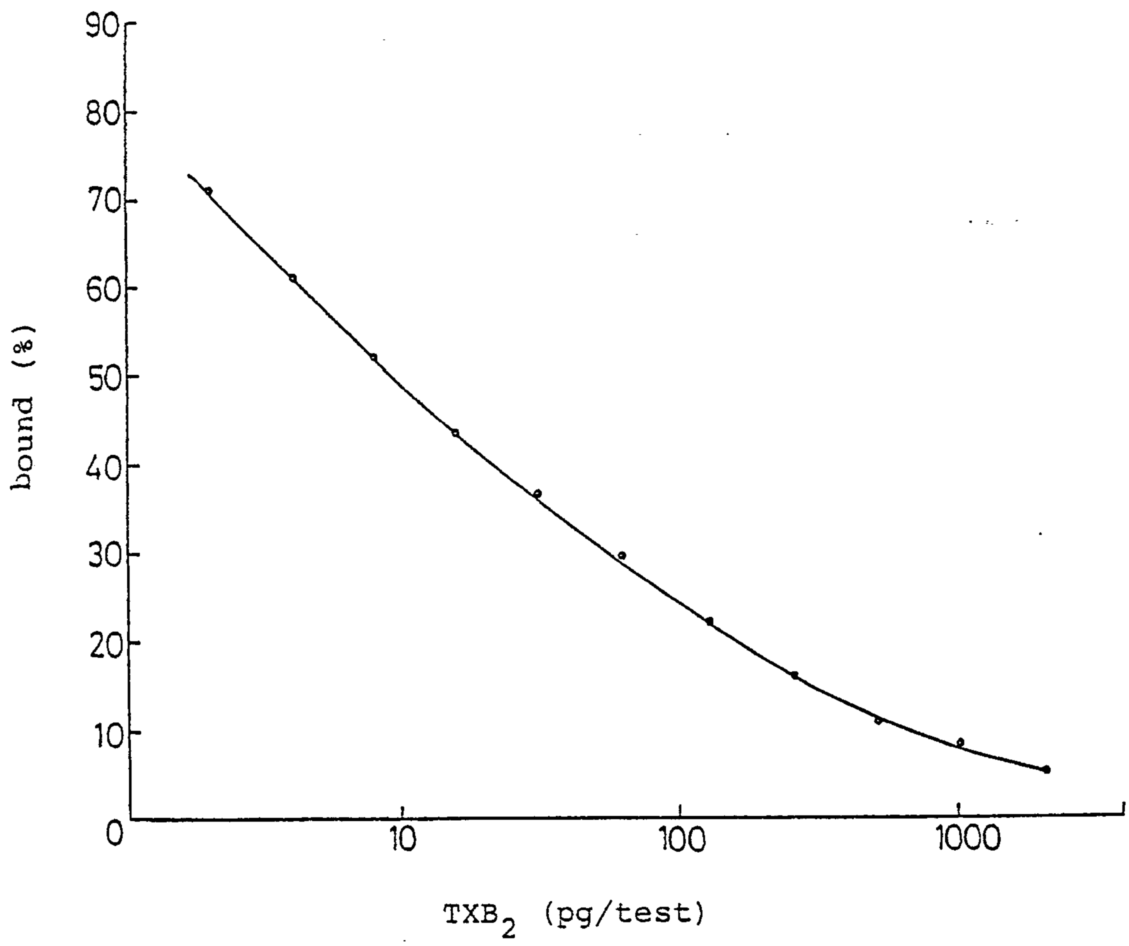

Figure 6

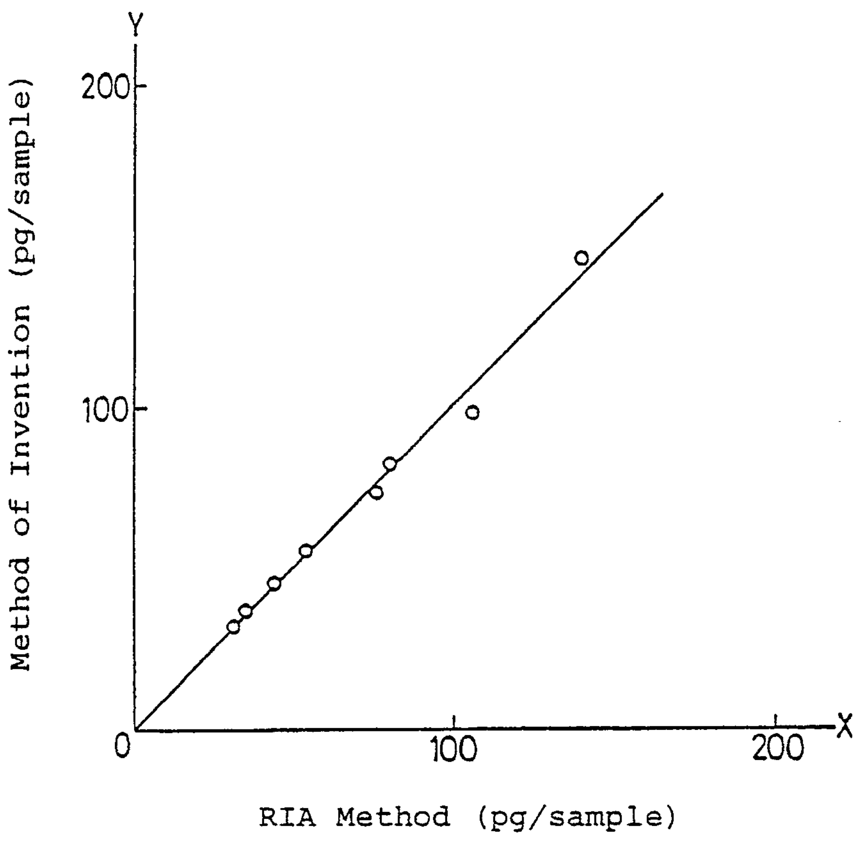
Y
200
100
0
100
200
X
Method of Invention (pg/sample)
RIA Method (pg/sample)

Figure 7

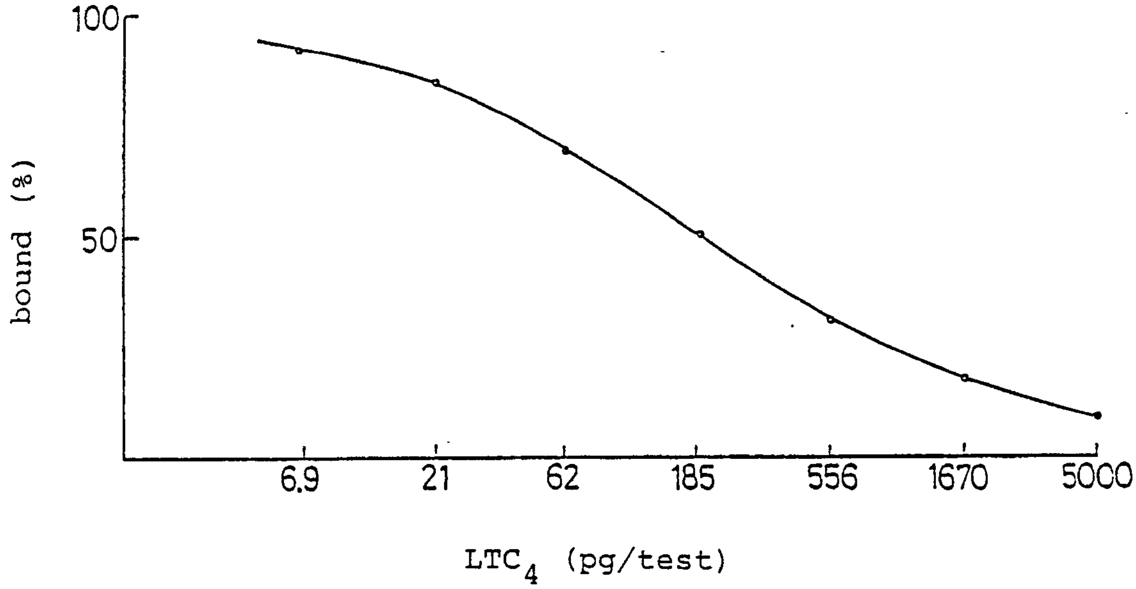

Figure 8

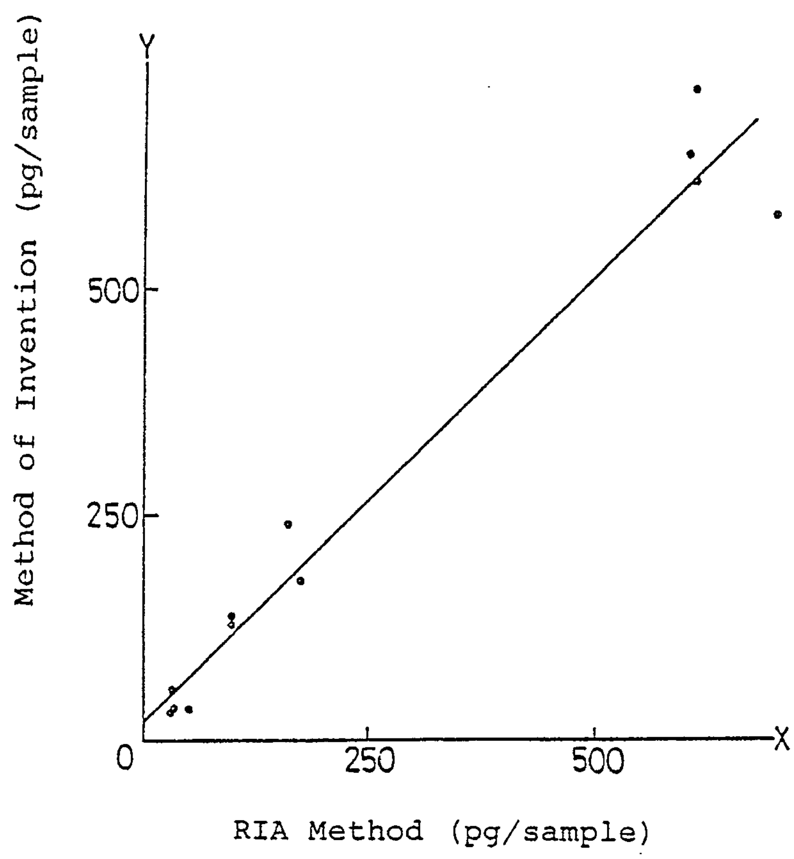
Y
500
250
0
250
500
X
Method of Invention (pg/sample)
RIA Method (pg/sample)